# EUROPEAN PATENT SPECIFICATION

(11) **EP 1 605 841 B1**
(45) Date of publication and mention of the grant of the patent: **20.06.2007**
(21) Application number: 04715649.2
(22) Date of filing: 27.02.2004
(51) Int. Cl.: A61B 17/30

(54) **APPARATUS FOR MAINTAINING CONTACT BETWEEN ELECTROPHYSIOLOGY ELEMENTS AND TISSUE AND SYSTEMS INCLUDING THE SAME**
VORRICHTUNG ZUR ERHALTUNG DES KONTAKTES ZWISCHEN ELEKTROPHYSIOLOGIEELEMENTEN UND KÖRPERGEWEBE UND SYSTEM DAFÜR
APPAREIL POUR MAINTENIR UN CONTACT ENTRE DES ELEMENTS ELECTROPHYSIOLOGIQUES ET UN TISSU, ET SYSTEMES COMPRENANT LEDIT APPAREIL

(30) Priority: 21.03.2003 US 395021
(43) Date of publication of application: 21.12.2005
(73) Proprietor: Boston Scientific Limited, St. Michael, Barbados, West Indies (BB)
(72) Inventor: SWANSON, David, K., Campbell, CA 94040 (US); PHAN, Huy, D., San Jose, CA 95127 (US)
(74) Representative: Schmitz, Jean-Marie
(86) International application number: PCT/US2004/005883
(87) International publication number: WO 2004/093698

(56) References cited:
- WO-A-97/10753
- US-A- 3 805 793
- US-A- 5 055 100
- US-A- 5 224 944
- US-A- 6 142 994
- US-A1- 2002 002 372

## Description

### BACKGROUND OF THE INVENTIONS

### 1. Field of Inventions

The invention relates to a suction device for use with an electrophysiology device that includes at least one operative element. Accordingly, the present invention relates generally to devices for performing diagnostic and therapeutic operations on body tissue.

### 2. Description of the Related Art

There are many instances where diagnostic and therapeutic elements (referred to herein collectively as "operative elements") must be positioned adjacent to body tissue. One instance involves the formation of therapeutic lesions to the treat cardiac conditions such as atrial fibrillation, atrial flutter and arrhythmia. Therapeutic lesions may also be used to treat conditions in other regions of the body including, but not limited to, the prostate, liver, brain, gall bladder, uterus and other solid organs. Typically, the lesions are formed by ablating tissue with one or more electrodes. Electromagnetic radio frequency ("RF") energy applied by the electrode heats, and eventually kills (i.e. "ablates"), the tissue to form a lesion. During the ablation of soft tissue (i.e. tissue other than blood, bone and connective tissue), tissue coagulation occurs and it is the coagulation that kills the tissue. Thus, references to the ablation of soft tissue are necessarily references to soft tissue coagulation. "Tissue coagulation" is the process of cross-linking proteins in tissue to cause the tissue to jell. In soft tissue, it is the fluid within the tissue cell membranes that jells to kill the cells, thereby killing the tissue. Depending on the procedure, a variety of different electrophysiology devices may be used to position a plurality of electrodes at the target location.

In recent years, devices such as surgical soft tissue coagulation probes that carry one or more diagnostic or therapeutic elements have been developed. These probes may be used, for example, in endocardial and epicardial procedures where access to the heart is obtained by way of a thoracostomy, thoracotomy or median stemotomy. Such probes also allow endocardial lesions to be formed as a secondary procedure during a primary open heart surgical procedure such as mitral valve replacement, aortic valve replacement, and coronary artery bypass grafting. In either case, it is frequently desirable to create continuous linear lesions for therapeutic purposes.

Tissue contact can be an issue in any electrophysiology procedure, including those which involve the use of surgical probes for diagnostic and therapeutic purposes. The failure to achieve and maintain intimate contact between the tissue and operative elements can result In gaps in what were intended to be continuous linear lesions. Such gaps may result in a failure to cure the patient's arrhythmia and atrial flutter or may create atrial flutter. Moreover, atrial flutter created by gaps in linear lesions can difficult to cure. Poor contact between the tissue and operative elements can also result in lesions that are not transmural. Lesion which are not transmural may, in turn, fail to cure the patient's arrhythmia or other medical condition. Another issue in electrophysiology procedures is operative element positioning and, more specifically, preventing the operative elements from moving after the physician has placed them adjacent to the target tissue region.

US-A-3805793 discloses an anastomotic apparatus for joining the open lumen end of a donor vessel to the side wall of a recipient vessel, comprising suction support means for supporting the open end of the donor vessel in contact with the side wall of the recipient vessel; selective actuable means for holding the support means and the open end of the donor vessel in sealed contact with the side wall of the recipient vessel without interrupting the flow of biological fluids in the recipient vessel; hollow lumen cutting means positioned within said support means for cutting through and removing a portion of the side wall of the recipient vessel circumscribed by the interior edge of the sealed end of the donor vessel; and selectively controlled suction means connected to the support means for securing the removed wall portion. This device also presents a plurality of longitudinally spaced ports, defined by a bifurcated passageway, ports which create a plurality of longitudinally spaced suction regions in the arched surfaces of the suction support means. A connector in the form of a clip secures the anastomotic device to the suction support means.

US-A-5224944 discloses a disposable aspirator assembly for use with a surgical handpiece, said surgical handpiece having a cylindrical handle portion which handle portion is grasped during use, and a surgical tip portion, comprising: an aspirator tip portion comprising an offset hollow tubular member having a central lumen at least a portion of which is dimensioned to enclose said surgical tip portion of said surgical handpiece; and an aspirator handle portion comprising an elongate cylindrical hollow tubular member having an open slot integrally formed in the aspirator handle portion and extending substantially the length of said aspirator handle portion for receiving the surgical handpiece handle.

US-A-5055100 a suction attachment for use with a handheld electrosurgical instrument or the like and for use with a source of low fluid pressure that has flexible vacuum tubing extending therefrom which instrument has a tubular handle with front and back ends thereof, said tube having a back end adapted for receiving said flexible vacuum tubing, an intermediate section proportioned to extend along the exterior surface of said instrument handle and a front end shaped to extend apart relationship therewith when said suction attachment is clamped on to said instrument handle with said intermediate section of said tube being directed along said handle surface, and clamping means for selectively clamping said intermediate section of said tube to said instrument handle.

### SUMMARY OF THE INVENTIONS

A suction device in accordance with a present invention includes the features of claim 1. Further embodiments of the invention are described in the dependent claims. The suction device may be used to convert electrophysiology devices that do not have suction capabilities into electrophysiology devices that do have suction capabilities.

The above described and many other features and attendant advantages of the present Inventions will become apparent as the inventions become better understood by reference to the following detailed description when considered in conjunction with the accompanying drawings.

### BRIEF DESCRIPTION OF THE DRAWINGS

Detailed description of preferred embodiments of the inventions will be made with reference to the accompanying drawings.
Figure 1 is a perspective view of an electrophysiology system in accordance with a preferred embodiment of a present invention.
Figure 2 is a plan view of a probe in accordance with a preferred embodiment of a present invention.
Figure 3 is a section view taken along line 3-3 in Figure 2.
Figure 4 is a section view taken along line 4-4 in Figure 2.
Figure 5 is an end view of the probe illustrated in Figure 2.
Figure 5A is a plan view of a probe in accordance with a preferred embodiment of a present invention.
Figure 5B is a section view taken along line 5B-5B in Figure 5A.
Figure 5C is a section view taken along line 5C-5C in Figure 5A.
Figure 6 is a top view of a suction device in accordance with a preferred embodiment of a present invention.
Figure 7 is a side view of the suction device illustrated in Figure 6.
Figure 8 is a bottom view of the suction device illustrated in Figure 6.
Figure 9 is a partial section view taken along line 9-9 in Figure 7.
Figure 10 is a section view taken along line 10-10 in Figure 8.
Figure 11 is a section view taken along line 11-11 in Figure 8.
Figure 12 is a section view taken along line 12-12 in Figure 11.
Figure 13 is a bottom view of showing a portion of the probe illustrated in Figures 2-5 secured to the suction device illustrated in Figures 6-12.
Figure 14 is a partial section view taken along line 14-14 in Figure 13.
Figure 15 is a top view of a suction device in accordance with a preferred embodiment of a present invention.
Figure 16 is a section view taken along line 16-16 in Figure 15.

### DETAILED DESCRIPTION OF THE PREFERRED EMBODIMENTS

The following is a detailed description of the best presently known modes of carrying out the inventions. This description is not to be taken in a limiting sense, but is made merely for the purpose of illustrating the general principles of the inventions.

The detailed description of the preferred embodiments is organized as follows:
I. Exemplary System Overview
II. Exemplary Surgical Probe System
III. Exemplary Suction System
IV. Exemplary Operative Elements, Temperature Sensing And Power Control
The section titles and overall organization of the present detailed description are for the purpose of convenience only and are not intended to limit the present inventions.

This specification discloses a number of structures, mainly in the context of cardiac treatment, because the structures are well suited for use with myocardial tissue. Nevertheless, it should be appreciated that the structures are applicable for use in therapies involving other types of soft tissue. For example, various aspects of the present inventions have applications in procedures concerning other regions of the body such as the prostate, liver, brain, gall bladder, uterus and other solid organs.

### I. Exemplary System Overview

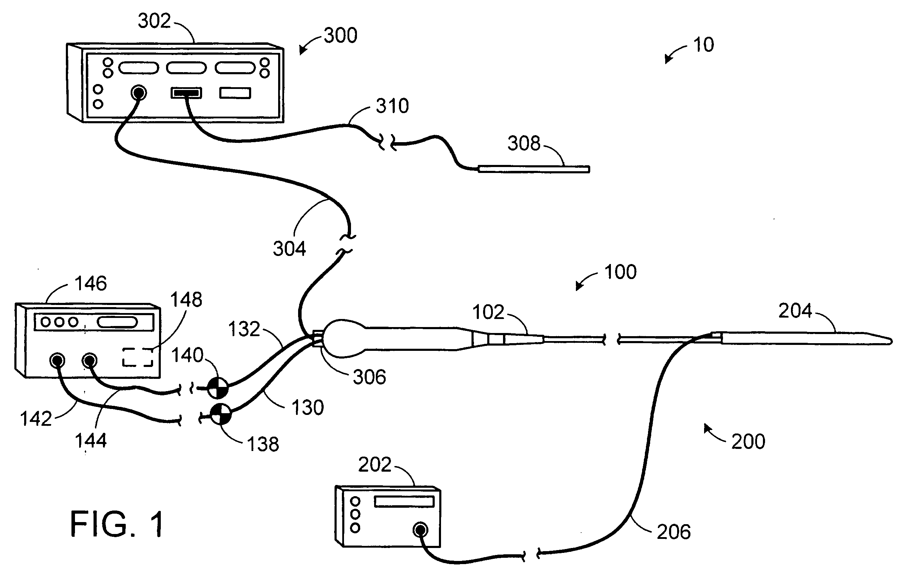

As illustrated for example in Figure 1, an electrophysiology system 10 in accordance with a preferred embodiment of a present invention includes a surgical probe system 100 and a suction system 200. The exemplary surgical probe system 100 includes a surgical probe 102. The exemplary suction system 200 includes a suction source 202 and a suction device 204 that may be removably secured to the distal portion of the surgical probe. When the suction source 202 is actuated, the suction device 204 will fix the position of the distal portion of the surgical probe 102 relative to the target tissue. The applied vacuum will also cause the tissue and operative elements carried by the surgical probe 102 to come into contact with one another. A power supply and control system 300 may be provided to supply power to the surgical probe 102.

There are a number of advantages associated with the exemplary electrophysiology system 10 generally and the suction device 204 in particular. For example, the suction device 204 may be used to convert a surgical probe such as the surgical probe 102, which does not have suction capabilities, into a surgical probe that does. The suction device 204 may also be used to convert other types of electrophysiology systems and devices, such as steerable and non-steerable diagnostic and/or therapeutic catheters, into a surgical probe with suction capabilities. Additionally, in those instances where the suction device 204 is initially provided with the surgical probe 102 or other electrophysiology device, the suction device can be easily removed so that the electrophysiology device may be utilized in low profile areas that are not large enough to accommodate the suction device.

### II. Exemplary Surgical Probe Structure

The exemplary suction system 200, which is described in greater detail in Section III below, may be used in combination with a wide variety of electrophysiology devices including, but not limited to, surgical probes, catheters, imaging devices, transducer arrays and diagnostic monitoring devices. Exemplary surgical probes and catheters are illustrated in U.S. Patent Nos. 6,142,994 and 6,287,301.

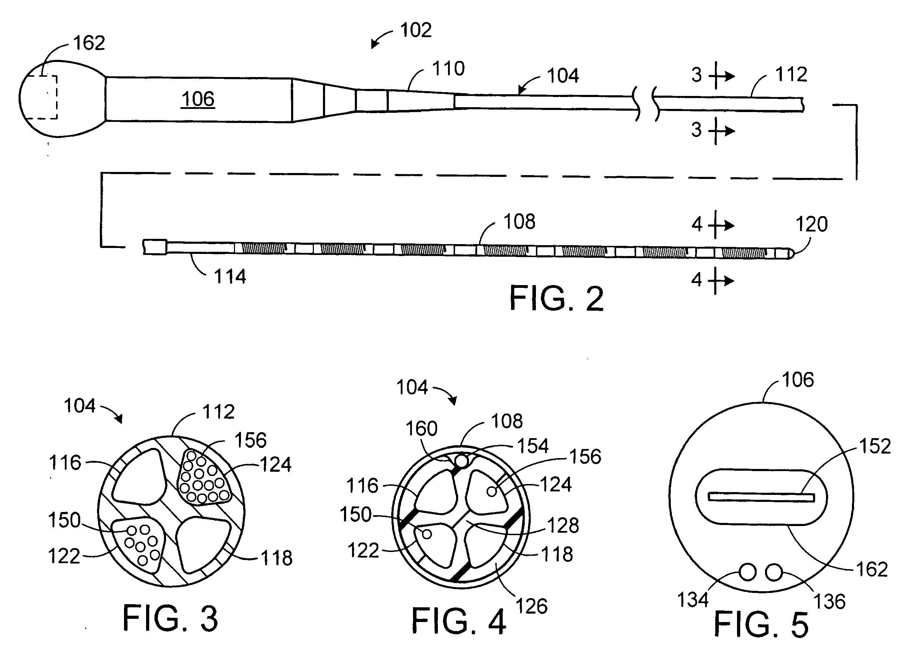

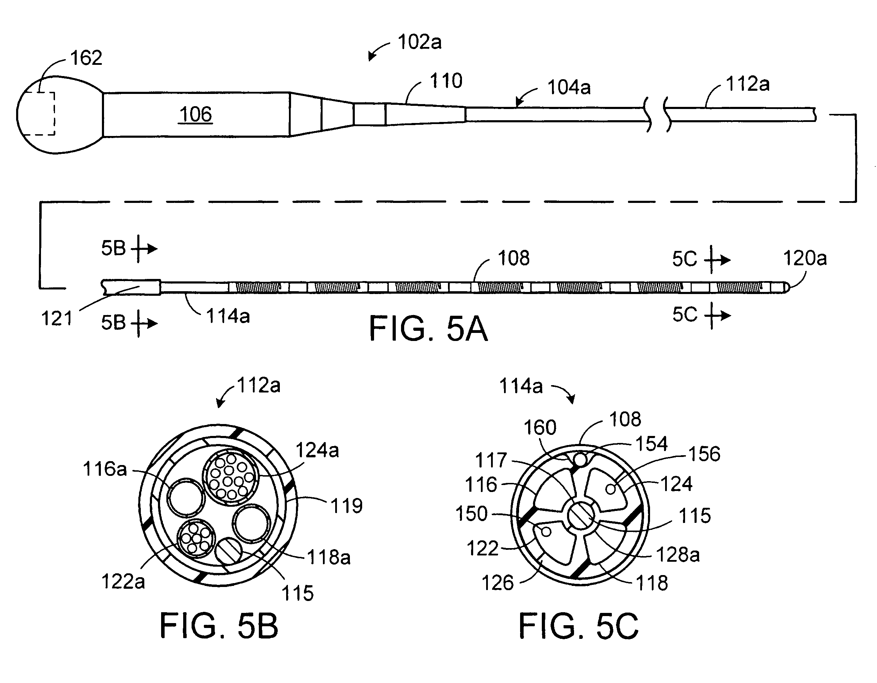

As illustrated for example in Figures 2-5, the surgical probe 102 in the exemplary surgical probe system 100 includes a shaft 104, a handle 106, and a plurality of electrodes 108 or other operative elements on the shaft. A strain relief element 110 may also be provided. The exemplary shaft 104 includes a proximal portion 112 and a distal portion 114. The proximal portion 112, which is relatively long (e.g. about 30 cm to 100 cm for cardiac treatment applications) and flexible, is secured to the handle 106. This allows the proximal portion 112 to be conveniently draped over the patient and beyond after the distal portion 114 and electrodes 108 have been positioned at the target tissue location. The distal portion 114, which carries the electrodes 108, is relatively short (e.g. about 2 cm to 15 cm for cardiac treatment applications) and is also flexible. [A probe with a malleable distal portion is discussed below with reference to Figures 5A-5C.] The shaft proximal and distal portions 112 and 114 may be a unitary structure or, alternatively, may be two separate structures that are secured to one another during assembly. The shaft proximal and distal portions 112 and 114 are also preferably formed from electrically non-conductive material.

The exemplary surgical probe system 100 is a cooled surgical probe system and, more specifically, the surgical probe system employs fluid to cool the electrodes 108 or other operative elements during coagulation procedures. As described in greater detail below, heat from the electrodes 108 is transferred to the fluid to cool the electrodes while energy is transferred from the electrodes to the tissue. Cooling the electrodes 108 during a coagulation procedure facilitates the formation of lesions that are wider and deeper than those that could be realized with an otherwise identical device which lacks the present cooling apparatus. Additionally, although gaseous cooling fluid may be employed, liquid is preferred.

Referring more specifically to Figures 3 and 4, the electrode cooling apparatus in the exemplary system 100 is composed primarily of the shaft distal portion 114 and fluid inlet and outlet lumens 116 and 118, which are formed in the proximal portion 112 as well as the distal portion. Heat from the electrodes 108 is transferred through the distal portion 114 to fluid that is flowing through the inlet and outlet lumens 116 and 118. Accordingly, in addition to being electrically non-conductive, the material used to form the distal portion 114 should be relatively high in thermal conductivity. As used herein, "relatively high" thermal conductivity is at least about 0.8 W/m•K and preferably ranges from about 0.8 to about 30 (or more) W/m•K. Suitable electrically non-conductive, thermally conductive thermoplastics for the distal portion 114 include flexible thermoplastic polymer materials, such as nylon or polyurethane, which are filled with a filler that promotes heat transfer. Suitable fillers include graphite, aluminum, tungsten and ceramic powders. Another suitable filler is Carborundum CarboTherm^{™} boron nitride powder manufactured by Saint-Gobain in Cavaillon, France. The proximal portion 112, on the other hand, does not have relatively high thermal conductivity and may be formed from, for example, flexible non-conductive thermoplastics such as such as Pebax^{®} material and polyurethane.

The inlet lumen 116 is connected to the outlet lumen 118 by a connection lumen (not shown) formed in a tip member 120 that is secured to the shaft distal portion 114 with adhesive or other suitable instrumentalities. The tip member 120 may be formed from, for example, two molded electrically non-conductive plastic parts. The tip member 120 also includes a pair of plugs (not shown) to seal the power and signal wire lumens 122 and 124. The power and signal wire lumens 122 and 124, as well as the power and signal wires 150 and 156 located therein, are discussed in greater detail in Section IV below. The tip member 120 may, alternatively, be replaced by a flexible tube that connects the inlet and outlet lumens 116 and 118. A pair of plugs would be provided for the power and signal wire lumens 122 and 124 when the flexible tube is employed.

In the exemplary implementation, where the shaft proximal and distal portions 112 and 114 are separate structures, the proximal portion may be larger in diameter than the distal portion because the proximal portion will be for the most part outside the patient. This configuration allows the cross-sectional areas of the fluid inlet and outlet lumens 116 and 118 within the proximal portion 112 to be maximized, thereby minimizing fluid flow resistance. There will be a step-down in the cross-sectional areas of the inlet and outlet lumens 116 and 118 where the proximal portion 112 is secured to the distal portion 114 in such a configuration. In the exemplary implementation, the outer diameter of the proximal portion 112 will be about 3 mm to about 5 mm, while the outer diameter of the distal portion 114 will be about 1.66 mm to 3.3 mm.

The exemplary shaft proximal and distal portions 112 and 114 are multi-lumen structures, each of which includes the fluid inlet and outlet lumens 116 and 118 and the power and signal wire lumens 122 and 124. Alternatively, a single lumen may be provided for the power and signal wires 150 and 156. The power and signal wire lumens may also be eliminated altogether in those instances where the power and signal wires 150 and 156 are sufficiently insulated and/or the cooling fluid is sufficiently non-conductive. Another alternative configuration is to arrange the lumens such that the power and signal wire lumens 122 and 124 are next to each other. Still another alternative configuration is a central cooling fluid inlet (or outlet) lumen that is connected to an outlet (or inlet) lumen that extends all, or essentially all, of the way around the outer structure. Yet another alternative is provide a tube with a relatively large inner lumen for the shaft proximal portion and series of smaller tubes within the tube to serve as the cooling fluid inlet and outlet lumens and the power and signal wire lumens. The smaller lumens may be connected to the fluid inlet and outlet lumens 116 and 118, as well as the power and signal wire lumens 122 and 124, in the shaft distal portion 114. Such an arrangement is discussed below with reference to Figures 5A-5C.

In addition to the aforementioned fillers, heat transfer may be promoted by minimizing the thickness of the electrically non-conductive material between the inlet and outlet lumens 116 and 118 and the electrodes 108 within the distal portion 114 and by maximizing the cross-sectional area of the inlet and outlet lumens within the distal and proximal portions of the shaft. With respect to the shaft distal portion 114 illustrated in Figure 4, for example, in an implementation where the outer diameter of the distal portion is about 8 French (2.66 mm), the thickness of the outer wall 126 between the electrode 108 and the inlet and outlet lumens 116 and 118 will be about 0.076 mm to about 0.356 mm. It should be noted that when the outer wall thickness is about 0.254 mm or less, materials with less than "relatively high" thermal conductivities, such as Pebax^{®} material and polyurethane, may also be used for the distal portion.

In order to allow the cooling fluid inlet and outlet lumens 116 and 118 to occupy as much of the cross-sectional area and circumferential area of the shaft 104 as possible, the power and signal wire lumens 122 and 124 should be just large enough to accommodate the power and signal wires 150 and 156. The width of the inlet and outlet lumens 116 and 118 (i.e. the distance between the outer wall 126 and the inner region 128) should be at least 2 times the thickness of outer wall and, preferably 4 times the thickness of the outer wall. In the implementation where the outer diameter of the distal portion 114 is about 8 French (2.66 mm), and the thickness of the outer wall 126 is about 0.102 mm to about 0.254 mm, the width of the inlet and outlet lumens 116 and 118 is preferably about 0.508 mm to about 1.02 mm.

As illustrated for example in Figure 1, fluid may be supplied to the surgical probe 102 by way of an infusion lumen 130, which is connected to the inlet lumen 116, and exit by way of a ventilation lumen 132, which is connected to the outlet lumen 118. The infusion and ventilation lumens 130 and 132 extend through a pair of apertures 134 and 136 in the handle 104 (Figure 5). The proximal ends of the infusion and ventilation lumens 130 and 132 are provided with on-off valves 138 and 140, which may be connected to the infusion and ventilation lines 142 and 144 of a fluid supply device 146 with a control system 148. An infusion pump capable of variable flow rates is one example of a suitable fluid supply device. The cooling fluid itself is not limited to any particular fluid. Preferably, however, the fluid will be a low or non-conductive fluid such as sterile water or 0.9% saline solution.

With respect to fluid temperature and flow rate, a suitable inlet temperature is about 0 to 25°C and the fluid supply device 146 may be provided with a suitable cooling system, if desired, to bring the temperature of the fluid down to the desired level. Although the fluid temperature will rise as heat is transferred to the fluid, the temperature will remain low enough to draw heat from the electrodes 108 as it flows through the inlet and outlet lumens 116 and 118. In a seven electrode embodiment such as those illustrated in Figures 1-5 where 150 W is being supplied to the electrodes 108, for example, a suitable constant fluid flow rate is about 5 ml/min to about 20 ml/min. In a closed system such as that illustrated in Figure 1 where the fluid is stored in the fluid supply device 146 and heated fluid is returned to the device, it has been found that a volume of fluid between about 10 and about 60 ml within the device will remain at room temperature (about 22°C) when the flow rate is between about 5 ml/min. and about 20 ml/min. Alternatively, in an open system where heated fluid is not returned to the fluid supply device 146, the device should include enough fluid to complete the procedure. 60 ml would, for example, be required for a 3 minute procedure where the flow rate was 20 ml/min.

Another exemplary surgical probe is generally represented by reference numeral 102a in Figures 5A-5C. Surgical probe 102a is a fluid cooled surgical probe that is substantially similar to the surgical probe 102 illustrated in Figures 1-5 and similar elements are represented by similar reference numerals. Here, however, the proximal portion 112a of the shaft 104a is flexible and the distal portion 114a is malleable. As used herein, a "malleable" object is an object that can be readily bent by the physician to a desired shape, without springing back when released, so that it will remain in that shape during the surgical procedure. Thus, the stiffness of a malleable object must be low enough to allow the object to be bent, but high enough to resist bending when the forces associated with the intended electrophysiology procedure.

In the exemplary embodiment illustrated in Figures 5A-6C, the proximal portion 112a is formed primarily by a flexible outer tube, while the distal portion 114a includes a malleable wire 115 that allows the physician to bend the distal portion into the desired shape. The distal portion 114a is provided with a central lumen 117 to accommodate the malleable wire 115. One end of the malleable wire 115 is mounted in the tip member 120a and the other end is soldered or otherwise secured to a relatively short (e.g. about 2 cm) hypotube 119 that is positioned within the distal end 121 of the proximal portion 112a. The proximal portion 112a also houses fluid inlet and outlet tubes 116a and 118a, which are connected to the fluid inlet and outlet lumens 116 and 118 in the distal portion 114a and to the infusion and ventilation lumens 130 and 132 in the handle 106, and power and signal wire tubes 122a and 124a, which are connected to the power and signal wire lumens 122 and 124 in the distal portion. Alternatively, the infusion and ventilation lumens 130 and 132 could simply extend all the way to the distal portion 114a for connection to the inlet and outlet lumens 116 and 118.

Additional details concerning fluid cooled surgical probes with both flexible and malleable distal sections may be found in U.S. Patent No. 6,939,350, which is entitled "Apparatus For Supporting Diagnostic and Therapeutic Elements In Contact With Body Tissue Including Electrode Cooling Device."

### III. Exemplary Suction System

As illustrated for example in Figure 1, and as noted above, the exemplary suction system 200 includes a suction source 202 and a suction device 204. The suction source 202 may be any suitable device that is capable of supplying the desired partial vacuum, which will typically range from about 300 mmHg to about 700 mmHg. The suction device 204, which is connected to the suction source 202 with a flexible suction tube 206, may be removably secured to the distal portion 114 of the surgical probe 102 (or to all or part of another electrophysiology device such as the distal portion of the surgical probe 102a). When the suction source 202 is actuated, the suction device 204 will affix itself to a tissue surface and hold the distal portion 114 of the surgical probe 102 in place relative to the tissue surface. Additionally, and depending on the rigidity of the suction device 204 and the rigidity of the tissue, the electrodes 108 will be brought into contact with the tissue surface when the suction source 202 is actuated because portions of the suction device will deflect, portions of the tissue surface will deflect, or portions of both the suction device and the tissue surface will deflect.

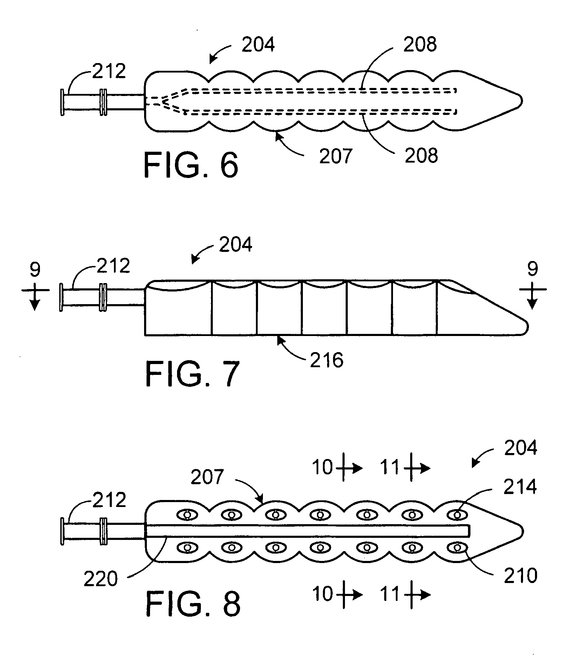

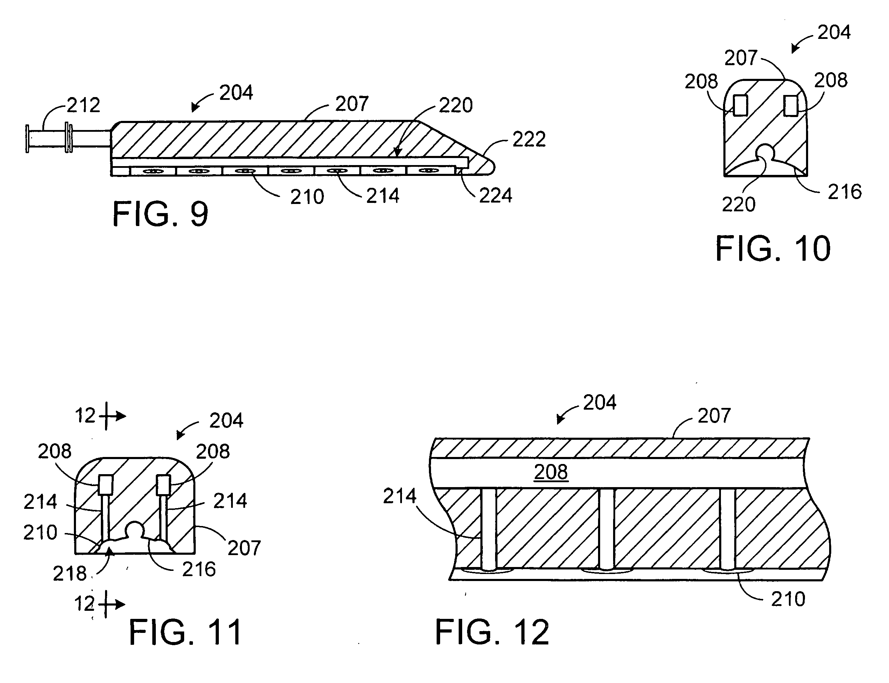

Turning to Figures 6-12, the exemplary suction device 204 includes a main body 207, a pair of internal suction lines 208 and a plurality of individual suction ports 210. The suction tube 206 may be connected to the internal suction lines 208 by a connector 212 such as, for example, the illustrated Luer connector. The suction ports 210 are respectively connected to the internal suction lines 208 by a plurality of apertures 214. The suction ports 210 are also formed in the curved bottom surface 216 (or "bottom wall") of the main body 207 and define respective suction regions 218 (Figures 10 and 11). During use, the curved bottom surface will form a seal with the tissue surface and air within the suction regions 218 will be drawn through the apertures 214, thereby causing the suction device 204 to adhere to the tissue surface.

The suction device 204 also includes a connector that enables it to be removably secured to the surgical probe distal portion 114 (or 114a or all or part of other.electrophysiology devices). Although the present inventions are not limited to any particular connector, the connector in the exemplary embodiment is a slot 220 into which the surgical probe distal portion 114 or 114a may be inserted. The slot 220 is generally semi-circular in cross-section and extend between about 180 to 360 degrees, and preferably about 300 degrees. The diameter of the slot 220 will preferably be about the same as the diameter of the surgical probe distal portion 114 or 114a. As such, the distal portion 114 or 114a may be removably snap fit into the slot 220. Additionally, once the surgical probe distal portion 114 or 114a is within the slot 220, it may be advanced distally toward the suction device nose 222 and into an aperture 224 for anchoring (Figure 9).

The specific size and shape of the suction device 204 will, of course, depend on the intended application, as will the choice of materials. Although the present inventions are not limited to any particular sizes, shapes or materials, one exemplary implementation that is especially well suited for cardiac treatment and use with the above-described surgical probe 102a is described hereafter. The suction device 204 is formed, preferably by molding, from a soft, flexible biocompatible material such as silicone rubber or urethane that is capable of withstanding temperatures up to 120 °C without melting or burning. When molded, the suction device 204 will be an integrally formed (i.e. one piece) structure, although some or all of the connector 212 may be added after molding depending on the type of connector employed. The overall length of the suction device 204, not including the connector 212, will be slightly longer than the shaft distal portion 114 or 114a, e.g. about 10 cm in an exemplary implementation where the distal portion is about 9 cm.

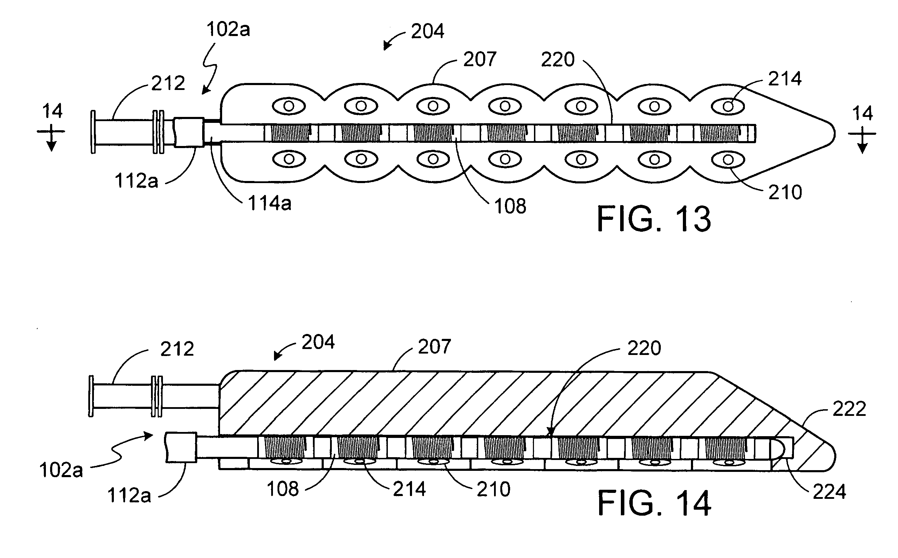

The exemplary suction ports 210 are generally concave and elliptical in shape and have a major diameter of about 5 mm, a minor diameter of about 3 mm, a depth of about 2 mm. In the illustrated embodiment, the spacing corresponds to the spacing of the electrodes on the associated probe. Alternatively, the exemplary elliptical (i.e. 5 mm x 3 mm x 2 mm) suction ports may be spaced apart by about 6 mm center-to-center. The distance between the bottom of the slot 220 and the bottom of the main body 207 is about 5 mm. This exemplary configuration will result in the surgical probe 102a and suction device 204 mating with one another in the manner illustrated in Figures 13 and 14. The surgical probe 102 and suction device 204 will mate with one another in a similar manner.

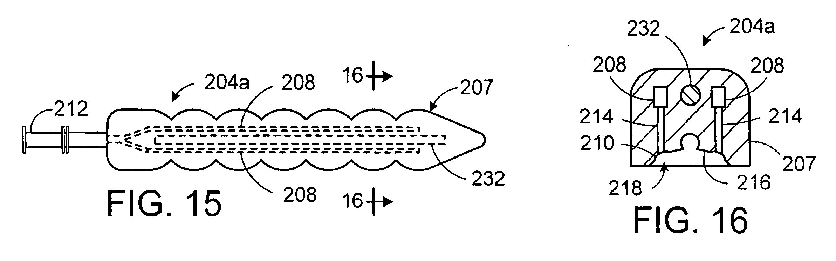

Another exemplary suction device is generally represented by reference numeral 204a in Figures 15 and 16. Suction device 204a is substantially similar to the suction device 204 and similar elements are represented by similar reference numerals. Here, however, suction device 204a is malleable and may be bent by the physician into a desired shape prior to being placed against tissue. Such a suction device is especially well suited for use with an electrophysiology device, such as surgical probe 102, with a flexible distal region. Of course, malleable suction devices may be used with malleable electrophysiology devices and flexible suction devices may be used with flexible electrophysiology devices.

In the illustrated embodiment, malleability is provided by a malleable wire 232 that may be molded into the suction device 204a. The malleable wire 232 should be strong enough to hold the remainder of the suction device 204a, which is preferably soft, flexible material, in the desired shape after bending. When suction is applied, the soft material associated with the suction regions 218 and/or the associated tissue will deflect in the manner described above. There will typically be little or no bending of the malleable wire 232.

### IV. Electrodes, Temperature Sensing And Power Control

In each of the illustrated embodiments, a plurality of spaced electrodes adapted to transmit RF energy are employed. However, operative elements such as such as lumens for chemical ablation, laser arrays, ultrasonic transducers, microwave electrodes, ohmically heated hot wires, single elongate flexible electrodes and the like may be substituted for the spaced electrodes.

Although the present inventions are not limited to any particular number, the exemplary probes 102 and 102a each include seven spaced electrodes 108. The spaced electrodes 108 are preferably in the form of wound, spiral closed coils. The coils are made of electrically conducting material, like copper alloy, platinum, or stainless steel, or compositions such as drawn-filled tubing (e.g. a copper core with a platinum jacket). The electrically conducting material of the coils can be further coated with platinum-iridium or gold to improve its conduction properties and biocompatibility. Preferred coil electrodes are disclosed in U.S. Patent No. 5,797,905 and 6,245,068.

Alternatively, the electrodes 108 may be in the form of solid rings of conductive material, like platinum, or can comprise a conductive material, like platinum-iridium or gold, coated upon the device using conventional coating techniques or an ion beam assisted deposition (IBAD) process. For better adherence, an undercoating of nickel, silver or titanium can be applied. The electrodes can also be in the form of helical ribbons. The electrodes can also be formed with a conductive ink compound that is pad printed onto a non-conductive tubular body. A preferred conductive ink compound is a silver-based flexible adhesive conductive ink (polyurethane binder), however other metal-based adhesive conductive inks such as platinum-based, gold-based, copper-based, etc., may also be used to form electrodes. Such inks are more flexible than epoxy-based inks. Open coil electrodes may also be employed.

The exemplary flexible electrodes 108 are preferably about 4 mm to about 20 mm in length. In the preferred embodiments, the electrodes are 12.5 mm in length with 1 mm to 3 mm spacing, which will result in an energy transmission region that is about 1 cm to about 14 cm in length and the creation of continuous lesion patterns in tissue when coagulation energy is applied simultaneously to adjacent electrodes. For rigid electrodes, the length of the each electrode can vary from about 2 mm to about 10 mm. Using multiple rigid electrodes longer than about 10 mm each adversely effects the overall flexibility of the device, while electrodes having lengths of less than about 2 mm do not consistently form the desired continuous lesion patterns.

With respect to operation, the exemplary electrodes 108 may be operated in a uni-polar mode, in which the soft tissue coagulation energy emitted by the electrodes is returned through an indifferent patch electrode (not shown) externally attached to the skin of the patient. Alternatively, the electrodes may be operated in a bi-polar mode, in which energy emitted by one or more electrodes is returned through other electrodes. Still another alternative is to supply power in the combined bi-polar/uni-polar mode described in U.S. No. 2004/162556 which is entitled "Power Supply And Control Apparatus And Electrophysiology Systems For.Use With Same." The amount of power required to coagulate tissue ranges from 5 to 150 w and depends on parameters such as set temperature and the flow rate of the fluid.

As illustrated for example in Figures 1-5C, the electrodes 108 in the exemplary probes 102 and 102a are electrically coupled to individual power wires 150 that conduct coagulating energy to them. The power wires 150 are passed in conventional fashion through the lumen 122 (or tube 122a) to a PC board 152 within the handle 104. Preferably, a plurality of temperature sensors 154 such as thermocouples or thermistors, may be located on, under, abutting the longitudinal end edges of, or in between, the electrodes 108. A reference thermocouple (not shown) may also be provided. In the exemplary implementation, temperature sensors 154 are located at both longitudinal ends of each electrode 108. The temperature sensors 154 are connected to the PC board 152 by signal wires 156 that pass though lumen 124 (or tube 124a).

In the exemplary embodiment, the temperature sensors 154 are preferably located within a linear channel 160 (Figures 4 and 5C) that is formed in the shaft distal portions 114 and 114a. The linear channel 160 insures that the temperature sensors will all face in the same direction (e.g. facing tissue) and be arranged in linear fashion. This arrangement results in more accurate temperature readings which, in turn, results in better temperature control. As such, the actual tissue temperature will more accurately correspond to the temperature set by the physician on the power supply and control device, thereby providing the physician with better control of the lesion creation process and reducing the likelihood that embolic materials will be formed. Such a channel may be employed in conjunction with any of the electrode support structures disclosed herein.

The power supply and control system 300 in the exemplary implementation illustrated in Figure 1 includes an electrosurgical unit ("ESU") 302 that supplies and controls power, such RF power. A suitable ESU is the Model 4810 ESU sold by Boston Scientific Corporation of Natick, Massachusetts. The ESU 302 transmits energy to the electrodes 108 and receives signal from the temperature sensors 154 by way of a cable 304 and a connector 306 arrangement. The connector 306 is configured to be inserted into a slot 162 (Figure 5) on the surgical probe handle 106 and to mate with the PC board 152.

The exemplary ESU 302 illustrated is operable in a bipolar mode, where tissue coagulation energy emitted by one of the electrodes 108 is returned through one of the other electrodes, and a unipolar mode, where the tissue coagulation energy emitted by the electrodes 108 is returned through one or more indifferent electrodes 308 that are externally attached to the skin of the patient with a patch, or one or more electrodes (not shown) that are positioned in the blood pool, and a cable 310. The exemplary ESU 302 is also configured to individually power and control each electrode 108. Suitable temperature sensors and RF power supply and control devices are disclosed in U.S. Patent Nos. 5,456,682, 5,582,609 and 5,755,715.

Although the present inventions have been described in terms of the preferred embodiments above, numerous modifications and/or additions to the above-described preferred embodiments would be readily apparent to one skilled in the art. Additionally, the scope of the inventions includes any combination of the elements from the various species and embodiments disclosed in the specification that are not already described. It is intended that the scope of the present inventions extend to all such modifications and/or additions and that the scope of the present inventions is limited solely by the claims set forth below.

## Claims

1. A suction device for use with an electrophysiology device that includes at least one operative element, the suction device comprising:
a plurality of longitudinally spaced suction ports (210) that create a plurality of longitudinally spaced suction regions (218); and
at least one connector (220) configured to removably secure longitudinally spaced portions of the electrophysiology device adjacent to the longitudinally spaced suction regions (218).

2. A suction device as claimed in claim 1, further comprising:
a suction line (208) configured to be connected to a suction source (202); and
a plurality of apertures (214) that respectively connect the suction line (208) to the plurality of suction ports (210).

3. A suction device as claimed in claim 1, wherein the suction ports (210) are flexible.

4. A suction device as claimed in claim 1, wherein the electrophysiology device defines a cross-sectional shape and the connector (220) defines a corresponding cross-sectional shape.

5. A suction device as claimed in claim 1, wherein at least a portion of the suction device is malleable.

6. A suction device as claimed in claim 1, further comprising:
a longitudinally extending malleable element (232).

7. A suction device as claimed in claim 1, wherein the suction ports (210) comprise elliptical suction ports.

8. A suction device as claimed in claim 1, wherein the plurality of longitudinally spaced suction ports comprises a plurality of longitudinally spaced pairs of suction ports (210) and the at least one connector (220) is positioned between the suction ports in each pair.

## Patentansprüche

1. Saugvorrichtung zur Verwendung mit einer elektrophysiologischen Vorrichtung, die mindestens ein wirksames Element beinhaltet, wobei die Saugvorrichtung umfasst:
eine Vielzahl in Längsrichtung beabstandeter Saugöffnungen (210), die eine Vielzahl in Längsrichtung beabstandeter Saugbereiche (218) erzeugen; und
mindestens ein Verbindungsstück (220), das konfiguriert ist, um in Längsrichtung beabstandete Teile der elektrophysiologischen Vorrichtung benachbart zu den in Längsrichtung beabstandeten Saugbereichen (218) lösbar zu befestigen.

2. Saugvorrichtung, wie in Anspruch 1 beansprucht, weiter umfassend:
eine Saugleitung (208), die zum Anschluss an eine Saugquelle (202) konfiguriert ist; und
eine Vielzahl von Öffnungen (214), die jeweils die Saugleitung (208) mit der Vielzahl von Saugöffnungen (210) verbinden.

3. Saugvorrichtung, wie in Anspruch 1 beansprucht, wobei die Saugöffnungen (210) flexibel sind.

4. Saugvorrichtung, wie in Anspruch 1 beansprucht, wobei die elektrophysiologische Vorrichtung eine Querschnittsform definiert und das Verbindungsstück (220) eine entsprechende Querschnittsform definiert.

5. Saugvorrichtung, wie in Anspruch 1 beansprucht, wobei mindestens ein Teil der Saugvorrichtung verformbar ist.

6. Saugvorrichtung, wie in Anspruch 1 beansprucht, weiter umfassend:
ein sich in Längsrichtung erstreckendes verformbares Element (232).

7. Saugvorrichtung, wie in Anspruch 1 beansprucht, wobei die Saugöffnungen (210) elliptische Saugöffnungen umfassen.

8. Saugvorrichtung, wie in Anspruch 1 beansprucht, wobei die Vielzahl in Längsrichtung beabstandeter Saugöffnungen eine Vielzahl in Längsrichtung beabstandeter Paare von Saugöffnungen (210) umfasst und das mindestens eine Verbindungsstück (220) zwischen den Saugöffnungen in jedem Paar positioniert ist.

## Revendications

1. Dispositif d'aspiration à utiliser avec un dispositif d'électrophysiologie qui englobe au moins un élément opérationnel, le dispositif d'aspiration comprenant :
plusieurs orifices d'aspiration (210) espacés en direction longitudinale qui génèrent plusieurs zones d'aspiration (218) espacées en direction longitudinale; et
au moins un raccord (220) configuré pour fixer de manière amovible des portions espacées en direction longitudinale du dispositif d'électrophysiologie en position adjacente aux zones d'aspiration (218) espacées en direction longitudinale.

2. Dispositif d'aspiration selon la revendication 1, comprenant en outre:
un conduit d'aspiration (208) configuré pour être raccordé à une source d'aspiration (202) ; et
plusieurs orifices (214) qui raccordent respectivement le conduit d'aspiration (208) auxdits plusieurs orifices d'aspiration (210).

3. Dispositif d'aspiration selon la revendication 1, dans lequel les orifices d'aspiration (210) sont flexibles.

4. Dispositif d'aspiration selon la revendication 1, dans lequel le dispositif d'électrophysiologie définit une configuration en section transversale et le raccord (220) définit une configuration correspondante en section transversale.

5. Dispositif d'aspiration selon la revendication 1, dans lequel au moins une portion du dispositif d'aspiration est malléable.

6. Dispositif d'aspiration selon la revendication 1, comprenant en outre :
un élément malléable (232) s'étendant en direction longitudinale.

7. Dispositif d'aspiration selon la revendication 1, dans lequel les orifices d'aspiration (210) comprennent des orifices d'aspiration elliptiques.

8. Dispositif d'aspiration selon la revendication 1, dans lequel lesdits plusieurs orifices d'aspiration espacés en direction longitudinale comprennent plusieurs paires d'orifices d'aspiration (210) espacées en direction longitudinale, ledit au moins un raccord (220) étant disposé entre les orifices d'aspiration dans chaque paire.
